Europäisches Patentamt

European Patent Office

Office européen des brevets

(19)

(11) **EP 1 310 557 A1**

(12) **EUROPEAN PATENT APPLICATION**

(43) Date of publication:
**14.05.2003 Bulletin 2003/20**

(51) Int Cl.7: **C12N 15/12**, C12N 15/13,
C07K 16/00, C07K 14/705

(21) Application number: **02080338.3**

(22) Date of filing: **13.09.1991**

---

(84) Designated Contracting States:
**AT BE CH DE DK ES FR GB GR IT LI LU NL SE**

(30) Priority: **14.09.1990 US 583626**

(62) Document number(s) of the earlier application(s) in
accordance with Art. 76 EPC:
**92900060.2 / 0 551 440**

(71) Applicants:
  • **THE TRUSTEES OF THE UNIVERSITY OF
    PENNSYLVANIA**
    **Philadelphia, PA 19104-3246 (US)**
  • **THE WISTAR INSTITUTE OF ANATOMY
    AND BIOLOGY**
    **Philadelphia, PA 19104 (US)**
  • **COLORADO STATE UNIVERSITY**
    **Fort Collins, CO 80522 (US)**

(72) Inventors:
  • **Weiner, David**
    **Penn Wynne Hills, PA 19151 (US)**

  • **Williams, Robert M.**
    **Fort Collins, CO 80521 (US)**
  • **Kieber-Emmons, Thomas**
    **Newtown Square, PA 19073 (US)**
  • **Williams, Wiliam**
    **Havertown, PA 19083 (US)**
  • **Greene, Mark I.**
    **Penn Valley, PA (US)**

(74) Representative: **Goodfellow, Hugh Robin et al
    Carpmaels & Ransford,
    43 Bloomsbury Square
    London WC1A 2RA (GB)**

Remarks:
This application was filed on 17 - 12 - 2002 as a
divisional application to the application mentioned
under INID code 62.

---

(54) **Design of bioactive peptides based on immunoglobulin structure**

(57)    The present invention relates to a method for producing peptides which have biological activity comprising: (i) identifying a region of a protein of interest which is homologous to a member of the immunoglobulin superfamily of molecules; (ii) synthesizing a peptide which comprises amino acid sequence found in the region of homology; and (iii) modifying the synthetic peptide such that it assumes a three-dimensional conformation which is biologically active. The invention is based, in part, on the discovery that a cyclic peptide comprising amino acid sequences of a CDR derived from an antibody directed toward a viral receptor molecule can be used to effectively inhibit the binding of virus to its receptor. The invention is also based in part on the discovery that a cyclic peptide corresponding to a portion of human immunodeficiency virus (HIV) gp120 that is homologous to immunoglobulins may be used to generate an anti-HIV immune response.

EP 1 310 557 A1

**Description**

| TABLE OF CONTENTS | | | | |
|---|---|---|---|---|
| | | | | **PAGE** |
| 1. | Introduction | | | 3 |
| 2. | Background of the Invention | | | 3 |
| 3. | Summary of the Invention | | | 4 |
| 4. | Description of the Figures | | | 5 |
| 5. | Detailed Description of the Invention | | | 7 |
| | 5.1 | Identification Of A Protein Region of Protein Which Shares Homology With A Member Of the Immunoglobulin Superfamily Of Molecules | | 7 |
| | 5.2 | Synthesis Of Peptides Of The Invention | | 12 |
| | 5.3 | Modification Of Peptides Of The Invention | | 13 |
| 6. | Example: Design Of A Cyclic Peptide Which Binds To The Cellular Reovirus Receptor And Can Block The Interaction Between Reovirus And Its Target Cells | | | 14 |
| | 6.1 | Materials and Methods | | 14 |
| | | 6.1.1. | Peptides | 14 |
| | | 6.1.2. | Reovirus | 14 |
| | | 6.1.3. | Monoclonal Antibodies | 14 |
| | | 6.1.4. | Determination Of Free Sulfhydryls In Peptides (Ellman Determination) | 15 |
| | | 6.1.5. | Radioimmunassay (RIA) | 15 |
| | | 6.1.6. | Competitive RIA | 15 |
| | | 6.1.7. | Inhibition of Viral Binding To Cells | 16 |
| | | 6.1.8. | Flow Cytometry Analysis | 16 |
| | | 6.1.9. | Coupling of Peptides To KLH And Immunization | 17 |
| | 6.2 | Results and Discussion | | 17 |
| | | 6.2.1. | Peptide Cyclization | 17 |
| | | 6.2.2. | Binding Of 9B.G5 To Peptides | 20 |
| | | 6.2.3. | Inhibition Of 9B.G5 - 87.92.6 Interaction By Peptides | 20 |
| | | 6.2.4. | Inhibition Of 9BG5 - Reovirus Type 3 Interaction By Peptides | 21 |
| | | 6.2.5. | Inhibition Of Reo3R - 87.92.6 Interaction by Peptides | 21 |
| | | 6.2.6. | Inhibition Of Reo3R - Reovirus Type 3 Interaction By Peptides | 21 |
| 7. | Example: Design Of An Immunogenic Human Immunodeficiency Virus Cyclic Peptide | | | 22 |

## 1. INTRODUCTION

The present invention relates to methods for designing peptides which have biological activity. According to the invention, functionally relevant regions of a protein may be identified by comparison of the protein's amino acid sequence with the amino acid sequences of members of the immunoglobulin superfamily of molecules. Synthetic peptides corresponding to homologous regions may then be chemically modified in order to produce molecules which exhibit biologically active three dimensional conformations.

## 2. BACKGROUND OF THE INVENTION

Advances in molecular biology have indicated that immunoglobulins, major histocompatibility complex antigens and T cell receptors are all members of a family of molecules referred to as the immunoglobulin superfamily (Oldstone, 1987, Cell 50:819-820). During evolution, it is likely that a single, useful gene duplicated, and its copies diverged to create related molecules with distinct functions. Accordingly, immunoglobulins, which are agents of humoral immunity; T cell receptors, which are associated with humoral as well as cellular immunity; and major histocompatibility complex molecules, involved in antigen presentation and the discrimination between self and nonself, all share homologies inherited from their common ancestor and exhibit related biological functions.

Of the members of the superfamily, the structure and function of immunoglobulins is best understood. Immunoglobulin molecules consist of a constant region and a variable region. The constant region is associated with cellular effector functions whereas the variable region participates in antigen recognition and binding.

Immunoglobulins of the most common class, IgG, consist of two heavy chains and two light chains linked together by noncovalent associations and also by covalent disulfide bonds. Each of the chains possesses a constant as well as a variable region. In the immunoglobulin molecule, the variable region is subdivided into framework regions, which are similar in structure among immunoglobulins, and hypervariable, complementarity determining regions (CDRs) which participate directly in antigen binding in the immunoglobulin active site.

X-ray crystallographic studies of purified immunoglobulin molecules have indicated that the active site is a crevice formed by the heavy and light chain variable regions, and that the dimensions of the active sites vary among immunoglobulin molecules consequent to amino acid sequence variations (Hood et al., 1978, in "Immunology," The Benjamin/Cummings publishing Co., Inc., Menlo Park, p. 208). Amino acid sequence, crystallographic structure, and specially designed hapten probes have been used in conjunction with computer analysis to elucidate the relationship between an immunoglobulin and the antigen which it recognizes.

## 3. SUMMARY OF THE INVENTION

The present invention relates to a method for producing peptides which have biological activity comprising:

(i) identifying a region of a protein of interest which is homologous to a region of a member of the immunoglobulin superfamily of molecules;
(ii) synthesizing a peptide which comprises amino acid sequence found in the region of homology; and
(iii) modifying the synthetic peptide such that it assumes a three-dimensional conformation which is biologically active.

The invention is based, in part, on the discovery that a cyclic peptide comprising amino acid sequences of a CDR derived from an antibody directed toward a viral receptor molecule can be used to effectively inhibit the binding of virus to its receptor. The invention is also based in part on the discovery that a cyclic peptide corresponding to a portion of human immunodeficiency virus (HIV) gp120 that is homologous to immunoglobulins may be used to generate an anti-HIV immune response.

Peptides designed using the methods of the invention may be used, for example, but not by way of limitation, to interfere with virus/receptor binding, to elicit an immune response, and to intervene in pathologic immune responses including autoimmunity and allergy.

In additional specific embodiments of the invention, the protein of interest may be an immunoglobulin molecule, and the method of the invention may comprise identifying a region of the molecule which is homologous to other immunoglobulins in that it structurally functions as a CDR; synthesizing peptides which comprise portions of the identified CDR, and then modifying the peptide such that it has biological activity. Example section 6 presents a nonlimiting working example of these embodiments, in which a cyclic peptide is designed to resemble a CDR of an antivirus receptor antibody.

## 4. DESCRIPTION OF THE FIGURES

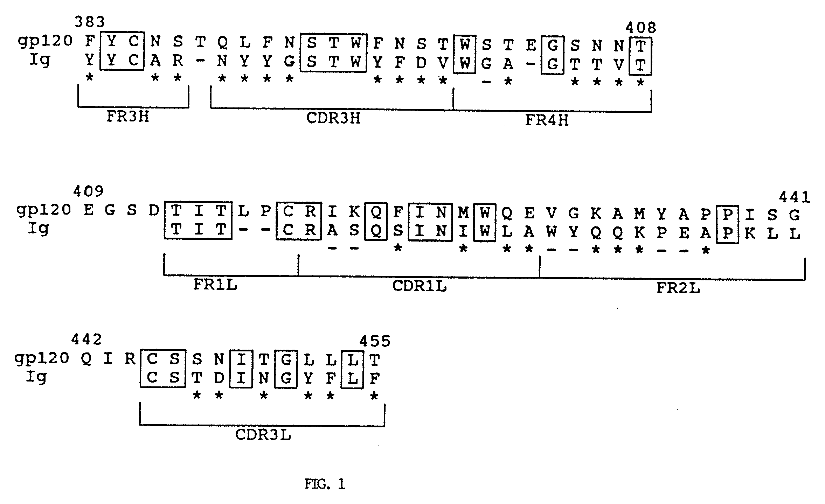

Fig. 1. Structural similarities in gp120 binding domain with Ig superfamily. Complementarity determining regions (CDR) and framework regions (FR) of the first, second, third and fourth domains of the respective heavy (H) or light (L) chains of several antibodies exhibited a degree of sequence homology with gp120 residues 383-455. The asterisks (*) mark residue positions of shared sequence homology between other HIV isolates and other antibodies. Crystallographic analysis of antibodies indicates that structural characteristics of CDR regions are preserved in spite of differences in sequence among antibodies. The dash (-) below a residue position denotes a lack of any sequence homology between an HIV isolated and an antibody. The dash (-) within a sequence denotes a deletion or insertion.

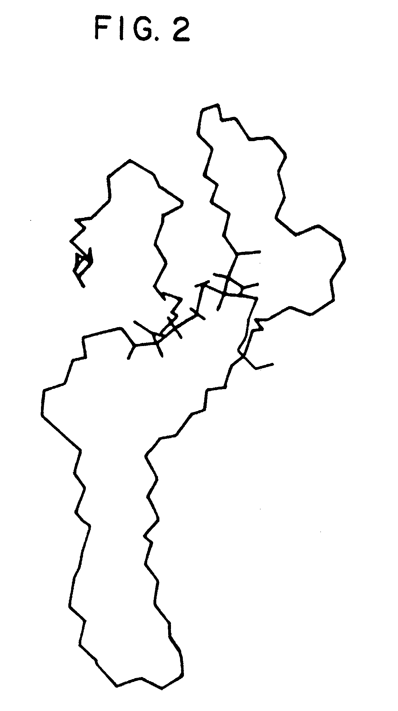

Fig. 2. Backbone representation of a proposed model for the putative binding side of gp120. The model extends from residue 413 through residue 456 (Fig. 1). The red loop highlights the analogous CDRI-like fold from

residues 419-429. The green loop highlights the CDR3L-like domain from residues 446-456. The disulfide bridge between residues 418 and 445 is in yellow. The white loop highlights residues 435-438. The red and green loops pack in a similar fashion to CDR1L and CDR3L of an antibody molecule with the disulfide positioned in an analogous manner.

Fig. 3. Sequence homology of CD4 and L3T4 with Ig light chains of known three-dimensional structure. Boxed areas highlight similar sequences. Dashes (-) indicate insertions/deletions. Sequence . alignment for comparative model building of CD4 utilizes a crystallographic template substituting the sequence of CD4 onto the homologous template. The choice of template is decided based upon the degree of sequence homology between a template and CD4 and the length of analogous turn/loop structures.

Fig. 4. Rate of loss of sulfhydryls for various peptides.

Fig. 5. Binding of 9B.G5 to peptides on solid phase RIA.

Fig. 6. Inhibition of 9B.G5 - 87.92.6 interaction by cyclic peptides.

Fig. 7. Inhibition of 9B.G5 - 87.92.6 interaction by cyclic peptides. Comparison with linear peptides derived from the 87.92.6 variable regions.

Fig. 8. Inhibition of 9B.G5 - reovirus type 3 interaction by cyclic peptides.

Fig. 9. Inhibition of 9B.G5 - reovirus type 3 interaction by cyclic peptides. Comparison with linear and dimeric peptides derived from the 87.92.6 variable regions.

Fig. 10. Inhibition of 87.92.6-Reo3R interaction by peptides.

Fig. 11. Specificity of $V_LC_9C_{16}$ peptide binding to the Reo3R.

Fig. 12. Inhibition of reovirus type 3 - Reo3R interaction by peptides.

Fig. 13. Comparison of cyclic and linear peptide interactions with the Reo3R by inhibition of $^{125}$I- reovirus type 3 binding.

Fig. 14. Comparison of binding of antisera resulting from immunization of rabbits with B138, 466, 1005-45, or 1029-04 peptides to gp120.

## 5. DETAILED DESCRIPTION OF THE INVENTION

The present invention relates to methods for designing peptides which have biological activity. For purposes of clarity of presentation, and not by way of limitation, the detailed description of the invention is divided into the following subsections:

(i) identification of a region of a protein which shares homology with a region of a member of the immunoglobulin superfamily of molecules;

(ii) synthesis of peptides of the invention; and

(iii) modification of peptides of the invention.

## 5.1. IDENTIFICATION OF A REGION OF A PROTEIN WHICH SHARES HOMOLOGY WITH A MEMBER OF THE IMMUNOGLODULIN SUPERFAMILY OF MOLECULES

According to the methods of the invention, the first step in the design of a bioactive peptide is the identification of a region in a protein of interest which shares homology with a region of a member of the immunoglobulin superfamily of molecules.

The protein of interest is a protein which is relevant to the biological process in which the peptide to be designed is active. For example, and not by way of limitation, if the desired peptide is to inhibit the attachment of a virus to its cellular receptor, the protein of interest may be a protein of the virus or it may be the cellular receptor. If the desired peptide is to be immunogenic, the protein of interest may be a protein comprised in the object of the intended immune response, preferably at its surface. For example, if the object of the intended immune response is a virus, then the protein of interest may be a protein which is found at the surface of the viral particle. In preferred embodiments of the invention, the protein of interest is a retroviral protein; in a specific embodiment, the protein of interest is the human immunodeficiency virus protein, gp120.

The determination of homology may be based on a comparison of amino acid or nucleic acid sequence, and/ or protein structure, between the protein of interest and a member of the immunoglobulin superfamily. Homology, as used herein, should be construed to refer to a similarity in sequence or structure shared by the protein of interest and the superfamily member which would be recognized by one skilled in the art as being greater than a reasonable artisan would expect to observe when comparing unrelated proteins. It may, in specific embodiments, be desirable to quantitate the percentage of identities in the sequences of the protein of interest and the superfamily member, such that the sequences are homologous if at least ten percent, and preferably at least twenty percent, of the compared residues in a region are identical. A region may be at least five residues in length. Alternatively, homology may be defined by the presence of similar molecular structures or geometries, such as β turns or loops.

Members of the immunoglobulin superfamily which may be used for comparison according to the invention include but are not limited immunoglobulins, major histocompatibility antigens, T-cell receptors, and any other related molecules which have been or will be identified. The sequences of these molecules may be derived experimentally using standard techniques or, preferably, may be obtained from a publicly accessible or other database, including, but not limited to, the Protein Sequence Database. Comparison of the sequences may be performed manually or, preferably, using a computer to identify similar or identical residues, sequences, or structures.

For example, and not by way of limitation, consider the following methods for the design of a peptide which may be used to elicit immunity toward HIV. HIV, the AIDS virus, enters its target cell in a series of steps. The first event in this sequence is the attachment of the viral envelope gp120 protein to CD4 on the surface of the target cell. Gp120 may be the protein of interest according to the invention. Computer graphics and comparative molecular modelling may be used to study the potential conformational properties of the CD4 binding site on gp120.

In the comparative modelling approach, the structure of an unknown protein is deduced from sequence similarities between portions of crystallographically known proteins and the protein fragment to be modelled (Greer et al., 1989, Prog. Clin. Biol. Res. 289:385-397). This approach has been used in the modelling of a wide range of proteins including antibodies and T cell receptors (De la Paz et al., 1986, EMBO J. 5:415-425; Chothia et al., 1988, EMBO J. 7:3745-3755). It has been hypothesized that retroviruses are evolving towards structurally mimicking epitopes of the immunoglobulin (Ig) superfamily (Oldstone, M.B.A., 1987, Cell 50:819-820) that interact with normal immune structures like CD4.

Structural examination of antigen combining regions of T cell receptors and antibodies indicates that the recognition sites of this particular superfamily class are organized β turns or loops. For MHC molecules, molecular recognition areas are highly a helical Bjorkman et al., 1987, Nature 329:506-512). Structural analysis of an anti-receptor antibody that mimics the cell attachment site of reovirus hemagglutinin affirms the possibility of shared β-type conformation as one underlying recognition feature bestowing mimicking properties on antibodies (Williams et al., 1988, Proc. Natl. Acad. Sci. U.S.A, 85:6488-6492). In this context, an anti-CD4 antibody that competes with gp120 for the same CD4 binding site is considered an antireceptor (anti-idiotypic) antibody that mimics gp120 (McDougal et al., 1986, Immunol. 137:2937-2944).

In the deduction of the possible topography for the CD4 binding site, one may first-examine the Protein Sequence Database (Devereux et al., 1984, Nucl. Acids Res. 12:387-395) using overlapping sequences of the BH10 isolate of HIV from residue 343 through 511. Optimal sequence alignment of the putative cell attachment site of gp120 with members of the Ig superfamily imply a degree of similarity between the site and antibody complementarity determining regions (CDRs) (Fig. 1).

The degree of similarity between the 383-455 region and CDR loops implies only that contact regions between gp120 and CD4 may exhibit β turns or loops, and not that gp120 itself folds like an immunoglobulin. This is evident because the intervening residues between the perceived β loops in gp120 are very different from those in antibodies. The disulfide bridge connecting residues 418 and 445 may preserve the anti-parallel β strands with a reverse turn geometry. In antibody structures, CDR1 and CDR3 of light chains pack against each other and are stabilized by a disulfide bond. If this type of structure exists in gp120, then the β loop comprising residues 419-429 would be packed with the β loop comprising residues 446-454; this packing may be stabilized by the disulfide bridge formed between amino acids 418 and 445. In this model, residues 430-438, representative of a β structure, would still be exposed for contacting CD4.

A molecular model for the region 413-455 (Fig. 2) may be constructed based upon these structural concepts, utilizing a light-chain antibody structure known as REI (Bernstein et al., 1977, Mol. Biol. 112:535-542) as a template for the first and third hypervariable region, in which the cysteines in gp120 at positions 418 and 445 form a disulfide bridge. The cysteines are positionally conserved with respect to each other as in the light chain. The model may be energy optimized using molecular mechanics and dynamics. The model depicted in Figure 1 indicates that the residues 421-438 define a central turn region of the domain that may be surface exposed for interaction with CD4. Neutralizing antibodies have been shown to be directed toward this site (Sun et al., 1989, J. Virol. 63:3579-3585). The in vitro biological activity of the 421-438 peptide fragment in modulating CD4-depending cellular function and this fragment's ability to induce an anti-HIV response is as described in Section 7, infra. This peptide was shown to block virus binding and appears to exhibit cellular regulatory functions such as immunosuppression that parallels gp120.

As an alternative example, CD4 may be the protein of interest.

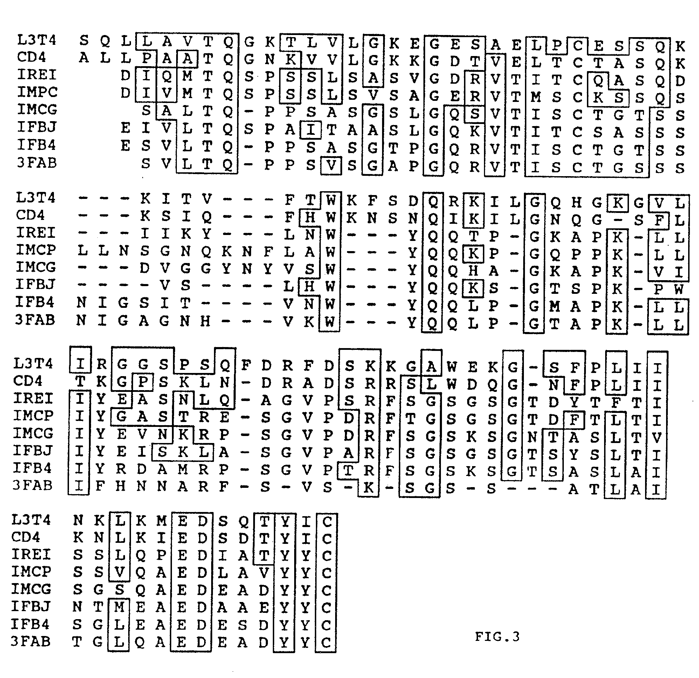

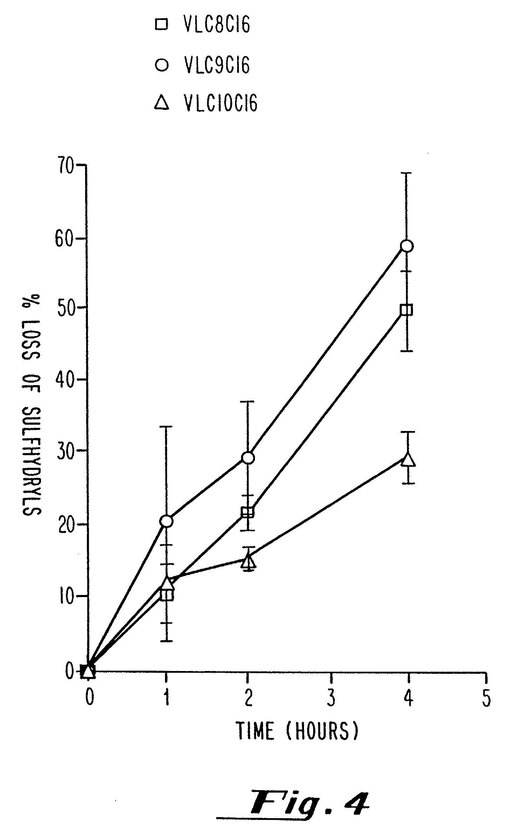

Interestingly, the CD4 receptor is only one of a small number of viral receptors that have been cloned (White et al., 1989, Cell 56:725-728). An examination of the DNA sequence and the exon/intron organization of the nucleotides which encode the CD4 glycoprotein strongly suggests that this gene has evolved from a primordial Ig gene (see Littman, D. R., 1987, Annu. Rev. Immunol. 5:561-584 for review). Homology profiles of the CD4 amino-acid sequence also reflect this relatedness to members of the Ig superfamily (Fig. 3; for reviews of the superfamily see Hood et al., 1985, Cell 40:225- 229 and Williams et al., 1988, Annu. Rev. Immunol. 6:381-405). The most striking homology between the CD4 protein and the Ig superfamily occurs between the first 84 amino acids of the CD4 glycoprotein and the Ig light chain variable domain (Fig. 3). Although other CD4 regions also show homology, secondary structure predictions generated from this domain of CD4 are also extraordinarily similar to those observed for the Ig light chain variable domains. Membership of the CD4 protein in the superfamily implies that the structure of the Ig-like domains of this protein should exhibit the classical "Ig-fold" (Schiffer et al., 1973, Biochemistry 12:4620-4631; Poljak et al., 1973, Proc. Natl. Acad. Sci. U.S.A. 70:3305-3310; Amzel et al., 1979, Annu. Rev. Biochem. 48:961-997). Therefore the alignment of CD4 with members of the Ig superfamily indicates tertiary structural similarities when residues, identified by X-ray diffraction studies of antibody hypervariable regions, are aligned on the premise that the basic building-block structure and interaction have been conserved (Fig. 4).

The sequence relationship between CD4 and immunoglobulins allows for general conceptions about the structure of CD4 to be formulated and correlated with CD4 epitope mapping studies. Analysis of Ig structure to delineate possible unique epitopes may therefore be used to examine gp120 binding to CD4. Structural analysis of epitope locations on the surfaces of antibodies suggests that there are separate or non-overlapping (epitope) recognition sites that involve both classical CDR and framework regions (Kieber-Emmons et al., 1986, Immunol. Rev. 90:29-48;Fig. 4). Such regions have been referred to as idiotype determining regions (IDR) (Kieber-Emmons et al., 1986, Immunol. Rev. 90:29-48). Each of these epitope (putative recognition) sites may have unique functional properties. By inference, the CD4/Ig superfamily sequence alignment implies that HIV, MHC class II, ancillary proteins such as CD3, and T cell receptors may bind to CD4 in noncompetitive ways. The relevance of the structural organization of the CD4 glycoprotein that influences gp120 binding will be discussed below.

## 5.2. SYNTHESIS OF PEPTIDES OF THE INVENTION

According to the present invention, the phrase "synthesis of peptides" is herein construed to refer to the production of peptides corresponding in sequence or structure to the homologous regions identified as set forth in 5.1, supra. These peptides may be produced using any method known in the art, including, but not limited to, chemical synthesis as well as biological synthesis in an in vitro or in vivo in a eukaryotic or prokaryotic expression system. The peptides may comprise the region of homology in addition to other sequences or may comprise only a portion of the region of homology.

## 5.3. MODIFICATION OF PEPTIDES OF THE INVENTION

Peptides of the invention prepared as described supra may be biologically active as produced or may require modification in order to assume a three-dimensional conformation which is biologically active. Modifications which may be performed, using standard techniques, according to the invention include but are not limited to cyclization, disulfide bond formation, glycosylation, phosphorylation, or the addition or subtraction of amino acid residues including amino acid residues which serve to produce a useful three dimensional conformation via a chemical linkage which is not generally found in natural peptides and/or mimetics including but not limited to, those described in Freidinger et al., 1980, Science 210;656; Hinds et al., 1988, J. Chem. Soc. Chem. Comm. 1447; Kemp et al., 1984, J. Org. Chem. 49: 2286; Kemp et al., 1985, J. Org. Chem. 50:5834; Kemp et al., 1988, Tetrahedron Lett. 29:5077; Jones et al., 1988, Tetrahedron Lett. 29:3853. An increase or decrease in bioactivity associated with modification may be ascertained using the appropriate assay system. For example, if the activity of the peptide is associated with immunogenicity, the ability of modified and unmodified peptide to elicit an immune response may be compared.

Further, if the desired geometry of a peptide is known, computer modelling may be used to identify modifications of the peptide which would result in the desired geometry. The success of these modifications in increasing bioactivity could then be evaluated using in vitro or in vivo assay systems.

In preferred specific embodiments of the invention, peptides are rendered cyclic by methods as set forth in example sections 6 and 7, infra. For example, cysteine residues within a peptide may be allowed to react with each other to form disulfide bonds.

## 6. EXAMPLE: DESIGN OF A CYCLIC PEPTIDE WHICH BINDS TO THE CELLULAR REOVIRUS RECEPTOR AND CAN BLOCK THE INTERACTION BETWEEN REOVIRUS AND ITS TARGET CELLS

### 6.1 MATERIALS AND METHODS

### 6.1.1. PEPTIDES

All peptides were synthesized by solid phase methods, deprotected and released from the resin utilizing anhydrous HF. Peptides were lyophilized and further purified by high performance liquid chromatography utilizing a TSK 3000 column and lyophilized. Purity was assessed by high performance liquid chromatography utilizing a C-6 column and a 0-70% acetonitrile gradient. All peptides were greater than 90% pure. Peptides (containing internal cysteine residues) were cyclized for experiments by dissolving them at 2 mg/ml in distilled water, and stirring them overnight exposed to the air. The peptides had no free sulfhydrlys following this procedure by Ellman determination.

### 6.1.2. REOVIRUS

Purified reovirus type 3 was prepared and radioodinated using methods set forth in Williams et al., 1988, Proc. Natl. Acad. Sci. USA 85:6488-6492, which is incoporated by reference in its entirety herein.

### 6.1.3. MONOCLONAL ANTIBODIES

Monoclonal antibodies 9BG5, which binds to reovirus type 3 hemagglutinin, and 87.92.6, which mimics the reovirus type 3 hemagglutinin by binding to both 9BG5 as well as the reovirus type 3 receptor, are as described in Williams et al., 1989, Proc. Natl. Acad. Sci. U.S.A. 86:5537-5541, also incorporated by reference in its entirety herein.

### 6.1.4. DETERMINATION OF FREE BULFEYDRYLS IN PEPTIDES (ELLMAN DETERMINATION)

Peptides dissolved in $dH_2O$ at 2 mg/ml were added at 5, 10, or 20μl to 10 mM $NaPO_4$pH7.0 for a final volume of lml. To this was added 6μl of 2,2'-bis azidothiobenzoic acid (ATBS, Sigma Chemical Co., St. Louis, MO) in 50 mM $NaPO_4$, pH 8.0. This was allowed to react for greater than 3 minutes, and the optical density (OD) at 420 nm was then determined.

### 6.1.5. RADIOIMMUNOASSAY (RIA)

RIA plates (Dynatech Laboratories, Alexandria, VA) were coated with peptides by evaporation of varying amounts of peptides in distilled water overnight at 37°C. The wells were washed with PBS, blocked with 2% bovine serum albumin (BSA) in PBS with 0.1% $NaN_3$, and washed with PBS. Partially purified 9BG5 [$(NH_4)_2SO_4$ precipitate] was added at varying dilutions for greater than 1 hour at 37°C. The wells were washed in PBS and 50,000 -100,000 counts per minute of $^{125}$I-labelled goat anti-mouse light chain (anti-k & anti-[Sigma] iodinated by chloramine T) was added per well in 1% BSA in PBS for 1-2 hours at 37°C. The wells were decanted, washed extensively, and CPM bound determined. Specific CPM bound was determined by subtracting the CPM bound to uncoated wells from the CPM bound to peptide coated wells.

### 6.1.6. COMPETITIVE RIA

RIA plates were coated with Staphylococcus protein A (Sigma Chemical Co., St. Louis, MO) by incubation of 50 μl per well of a 5 μg/ml solution overnight at 4°C. The wells were washed with PBS, blocked with 2% BSA/PBS/ 0.1%$NaN_3$, and purified 9BG5 or isotype matched control monoclonal (All) adsorbed to the wells by incubation of 50 μl of a 10 μg/ml solution (purified antibody) in 1% BSA/PBS/$NaN_3$ for 1-2 hours at 37°C. The wells were washed, and competitors were added at various concentrations in 100 μl of 0.5% BSA/0.45% NaCl/0/05% phosphate buffer pH7. 2 for 1 hour at 37 C. $^{125}$I-labelled reovirus type 3 (5-10x10[5] CPM per well) or unlabelled antibody (87.92.6 or isotype matched monoclonal E4.49.2) at a 1:100 dilution of ascites in 1% BSA/PBS/0.1% $NaN_3$) was added for an additional 30-45 minutes at 23°C. For wells incubated with 87.92.6, the wells were washed in PBS, and $^{125}$I-labelled goat anti-mouse Ig added for an additional 60 minutes at 37°C. The wells were washed extensively, and CPM bound determined. For reovirus binding, specific CPM bound was determined by subtracting CPM bound to All coated wells from CPM bound to 9BG5 coated wells. For 87.92.6 binding, specific binding was determined by subtracting CPM bound following incubation with E4.49.2 ascites from CPM bound following 87.92.6 incubation. % inhibition binding was determined by the formulae:

[(Specific CPM bound w/o Inhibitor) - (Specific CPM bound with inhibitor) x 100]

divided by specific CPM bound without inhibitor.

### 6.1.7. INHIBITION OF VIRAL BINDING TO CELLS

The cells were centrifuged and washed twice in 1% BSA/PBS/0.1% $NaN_3$, and 5X10[4] cells or 1.25x10[6] R1.1

cells in 50 ul distributed in 2%BSA/PBS/NaN$_3$ blocked RIA wells. For peptide studies, 50 µl of inhibitor was added in dH$_2$O to the cells. Following a 30 minute incubation, L cells and $^{125}$I-labelled reovirus type 3 were combined for an additional 30 minutes at 37°C. The cells were spun, washed 3x in ice cold PBS, and specific CPM bound was determined as noted above. Percent inhibition of binding was calculated by the formulae noted above.

### 6.1.8. **FLOW CYTOMETRY ANALYSIS**

The ability of peptides to inhibit antibody binding to cells was determined by preincubation of the cells with varying amounts of inhibitor (in 100 µl dH$_2$O) for 30 min. -1 hour at 23°C. Cells (either L cells or R1.1 cells) were washed in 1% BSA/PBS/0.1% NaN$_3$, and resuspended at 10$^7$/ml. 100 µl of cells were then added in 1%BSA/PBS/0.1% NaN$_3$, and the incubation continued for 20-30 minutes. Antibodies (5 or 10 µl were added for an additional 20 minutes at 23°C. Ice cold 1% BSA/PBS/0.1% NaN$_3$, was added, the cells centrifuged and washed prior to counterstaining with a 1:100 dilution of FITC goat anti-mouse Ig (Fisher Scientific) in 1% BSA/PBS/0.1% NaN$_3$. The cells were washed twice and fluorescence intensity determined. Inhibition of binding was calculated as noted above with mean channel number utilized in place of CPM.

### 6.1.9. **COUPLING OF PEPTIDES TO KLH AND IMMUNIZATION**

Peptides were coupled by gluteraldehyde fixation or specific coupling through a heterobifunctional crosslinker (MBS, Pierce Chemical Co.) (Romano et al., 1989, J. Neurochem. 53:362-369). Immunization was as described in Romano et al., supra.

### 6.2. **RESULTS AND DISCUSSION**

### 6.2.1. **PEPTIDE CYCLIZATION**

One measure of the optimal folding conformation of VL peptide is reflected by the ability of cysteine-containing variates to cyclize. If the cysteine residues are placed in various positions in one or the other side of a predicted reverse turn, the residues placed in the most energetically favorable locations for assuming a reverse turn structure should also cyclize most rapidly. This can be established utilizing several cysteine containing peptides as outlined in Table I.

These peptides were subjected to oxidation by agitating a solution (2 mg.ml in 0.1 $\underline{M}$ NaHCO$_3$) at 37 C for varying periods of time exposed to air. The disappearance of free sulfhydryls was quantitated by Ellman determination as above, and % loss of sulfhydryls with time calculated. The results are shown in Figure 4.

Peptides (Table I) were agitated at 37°C for varying periods of time and loss of sulfhydryls quantitated. As noted, $V_LC_6C_{16}$ and $V_LC_9C_{16}$ had the most rapid loss of sulfhydryls in this assay, while $V_LC_{10}C_{16}$ peptide forms intramolecular disulfide bridges more slowly than the other two peptides, and implies that the corresponding cyclic conformation of $V_LC_{10}C_{16}$ may be enregetically more costly to assume than that of $V_LC_8C_{16}$ or $V_LC_9C_{16}$.

The oxidation of these peptides did not necessarily imply that cyclic peptide formation was taking place, as intermolecular disulfide bridges also might have been forming. This issue was also examined by examining reduced (2-mercaptoethanol treated) and -non-reduced variates of these peptides by size-exclusion chromatography utilizing a sephadex G-10 superfine column. These studies indicated that both $V_LC_8C_{16}$ and $V_LC_9C_{16}$ peptides remained chiefly as monomers following oxidation, while a sizeable proportion of $V_LC_{10}C_{16}$ migrated more rapidly following oxidation. This indicates that the $V_LC_{10}C_{16}$ is forming intermolecular disulfide bridges, with subsequent formation of higher molecular weight forms. In contrast, $V_LC_8C_{16}$ and $V_LC_9C_{16}$

did not form intermolecular disulfide bridges, indicating that these peptides more readily fold into an appropriate conformation for intramolecular disulfide bond formation.

TABLE I Peptides Utilized in These Studies

Designation        Sequence

$V_L$:          Lys Pro Gly Lys Thr Asn Lys Leu Leu Ile Tyr Ser Gly Ser Thr Leu Gln

$V_L$SH: Cys Lys Pro Gly Lys Thr Asn Lys Leu Leu Ile Tyr Ser Gly Ser Thr Leu Gln

$V_L C_8 C_{16}$:   Lys Pro Gly Lys Thr Asn Lys Cys Leu Ile Tyr Ser Gly Ser Thr Cys Gln

$V_L C_9 C_{16}$:   Lys Pro Gly Lys Thr Asn Lys Leu Cys Ile Tyr Ser Gly Ser Thr Cys Gln

$V_L C_{10} C_{16}$:  Lys Pro Gly Lys Thr Asn Lys Leu Leu Cys Tyr Ser Gly Ser Thr Cys Gln

B138:       Lys Gln Phe Ile Asn Met Trp Gln Glu Val Gly Lys Ala Met Tyr Ala Pro Pro

1005/45:    Cys Arg Ile Lys Gln Phe Ile Asn Met Trp Gln Glu Val Gly Lys Ala Met Tyr Ala Pro Pro
            Ile Ser Gly Gln Ile Arg Cys

### 6.2.2. BINDING OF 9B.G5 TO PEPTIDES

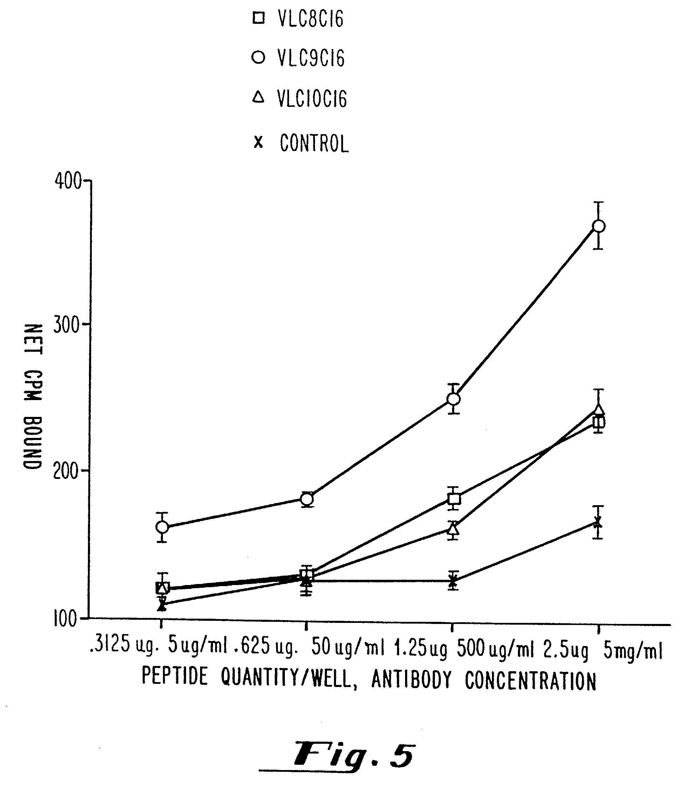

To assess the optimal conformation for binding of the $V_L$ peptide analogs, they were utilized to coat radioimmunoassay (RIA) plates, and 9B.G5 bound by standard RIA procedures. The results are shown in Figure 5.

As can be seen, binding to $V_LC_9C_{16}$ peptide was higher than binding to the other cyclic $V_L$ peptide analogs. This indicates that $V_LC_9C_{16}$ peptide has enhanced binding to 9B.G5 on solid phase RIA in comparison with the other cyclic peptides.

### 6.2.3. INHIBITION OF 9B.G5 - 87.92.6 INTEFACTION BY PEPTIDES

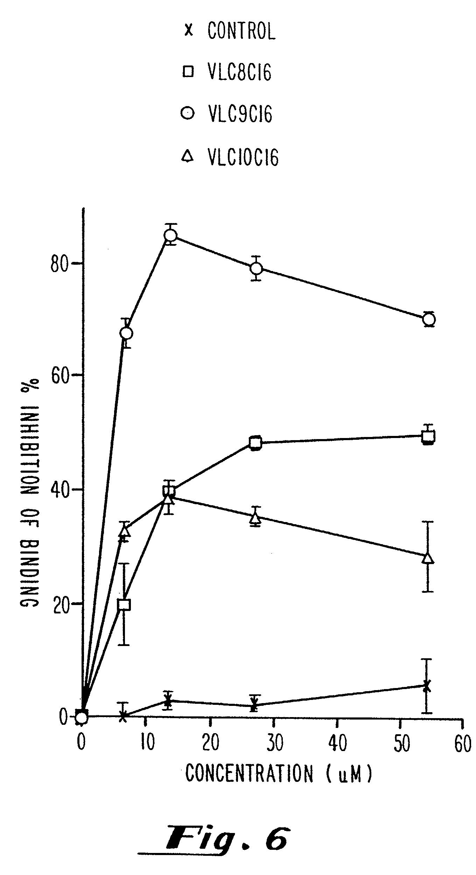

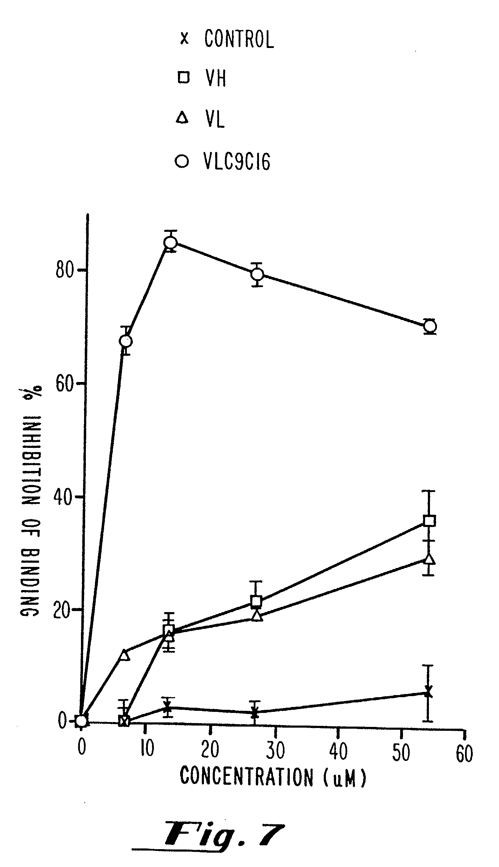

While the solid phase RIA indicates a higher affinity of 9B.GS to $V_LC_9C_{16}$ peptide compared to the other cyclic peptides, it does not address the affinity of this interaction in solution. To investigate the optimal solution conformation for 9B.G5 binding, the peptides were utilized to inhibit 9B.G5 - 87.92.6 interaction in a liquid phase assay. As shown in Figure 6, the results of this assay indicate the $V_LC_9C_{16}$ peptide again demonstrates a higher affinity of interaction compared with the other cyclic peptide variants. When compared with a linear analog of $V_L$ peptide (Figure 7), $V_LC_9C_{16}$ peptide displays an increased affinity of binding (40 fold higher affinity). This indicates that the increased conformational stability of this cyclic peptide increases its binding affinity for 9B.G4. It is also demonstrated that a peptide derived from the 81.92.6 heavy chain variable region CDR II ($V_H$ peptide) is able to inhibit the 87.92.6 - 9B.G5 interaction. While this peptide inhibits the idiotype- anti-idiotype interaction, it does not significantly interact with the reovirus type 3 receptor (Reo3R).

### 6.2.4. INHIBITION OF 9B.GS-REOVIRUS TYPE 3 INTERACTION BY PEPTIDES

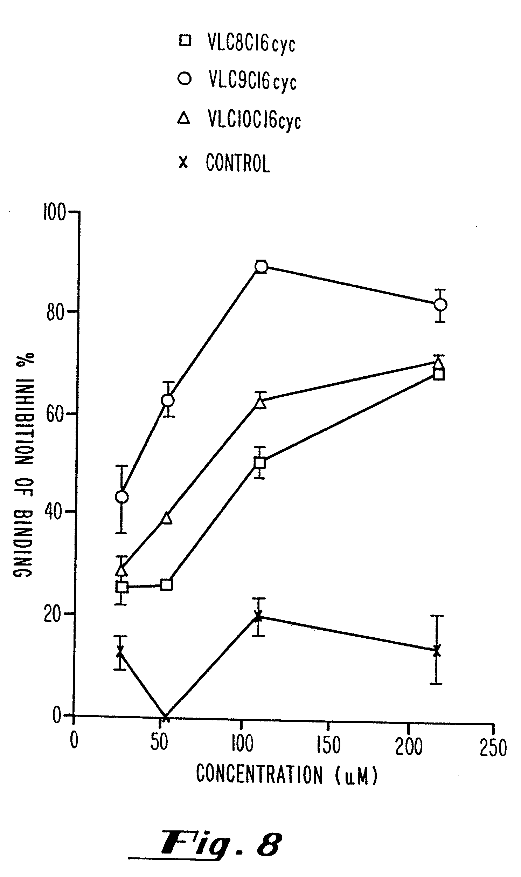

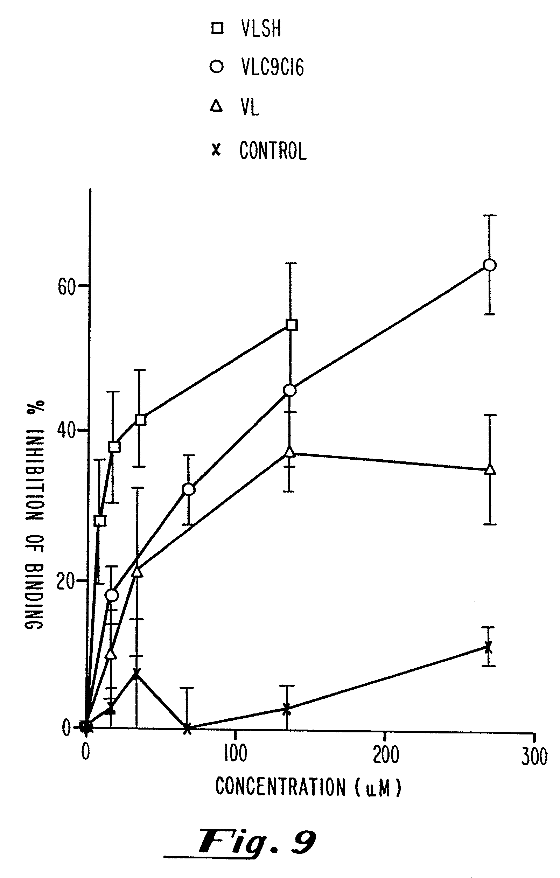

To confirm that $V_LC_9C_{16}$ peptide represents an optimal conformation for 9B.G5 binding in solution phase it was utilized to inhibit binding of $^{125}$I-labelled reovirus type 3 to 9B.G5 in a similar assay. The results are shown in Figure 8. As can be seen, $V_LC_9C_{16}$ peptide also exhibited higher affinity than the other cyclic peptides in this assay. When compared with linear $V_L$ peptide and dimeric $V_L$SH peptide (Figure 9), $V_LC_9C_{16}$ peptide demonstrates higher affinity than linear $V_L$ peptide, and similar affinity on a molar basis as dimeric $V_L$SH peptide.

### 6.2.5. INHIBITION OF REO3R-87.92.6 INTERACTION BY PEPTIDES

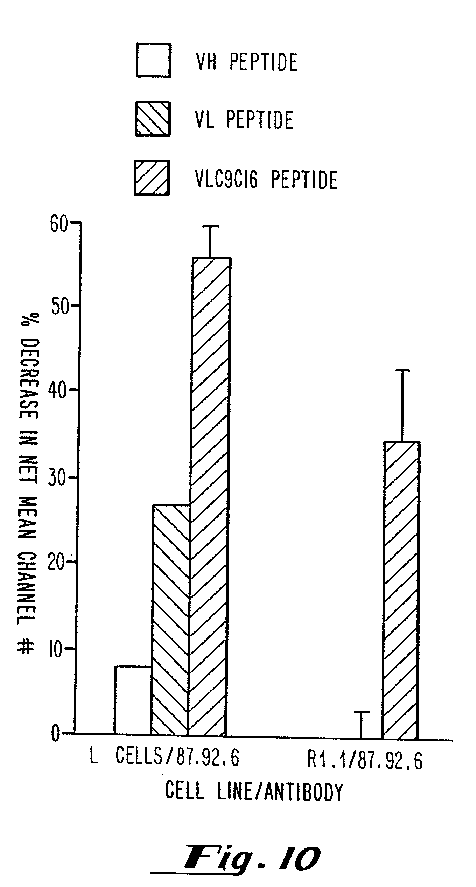

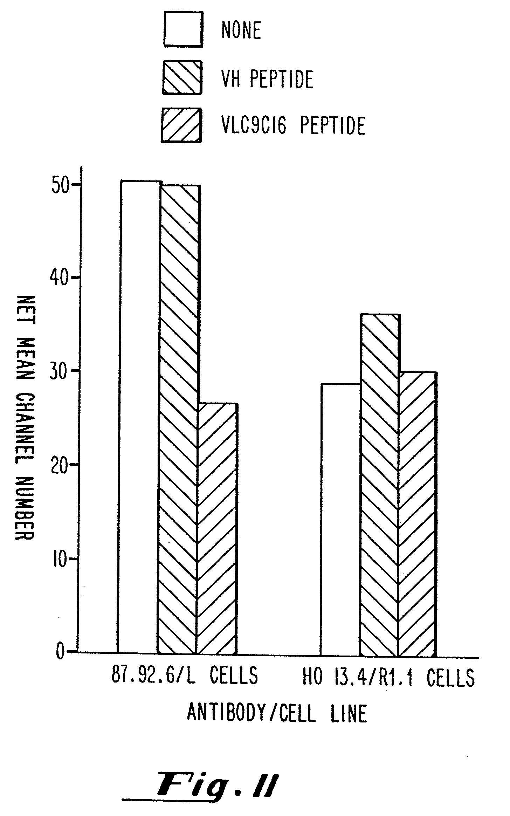

To assess the affinity of the cyclic peptides for the reovirus type 3 receptor (Reo3R), they were utilized in a series of assays to inhibit binding of 87.92.6 or control antibodies to specific receptors. As shown in Figure 10, $V_LC_9C_{16}$ peptide inhibited binding of 87.92.6 to murine L cells and R1.1 thymoma cells. In contrast, linear $V_H$ peptide had no effect on 87.92.6 binding, while $V_L$ peptide is a less effective competitor on L cells, and ineffectual on R1.1 cells. The inhibition by $V_LC_9C_{16}$ peptide was specific as binding of isotype matched monoclonal H013.4 to Thy1.2 molecules was not inhibited by $V_LC_9C_{16}$ peptide (Figure 11). Thus, $V_LC_9C_{16}$ peptide is a specific Reo3R ligand with enhanced affinity compared with its linear analog.

### 6.2.6. INHIBITION OF REO3R-REOVIRUS TYPE 3 INTERACTION BY PEPTIDES

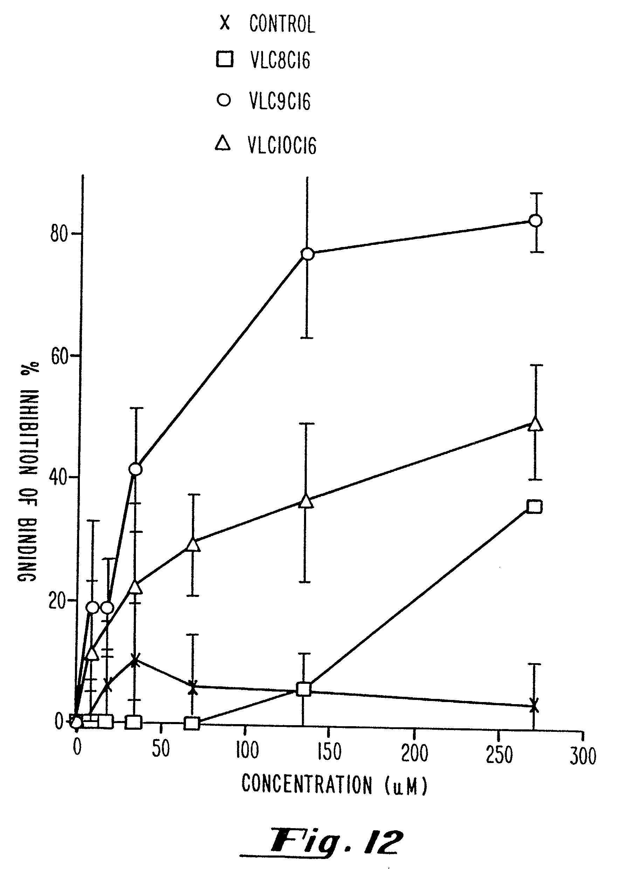

To further evaluate the interaction of $V_LC_9C_{16}$ peptide with the Reo3R, the peptide was utilized to compete with $^{125}$I-labelled reovirus type 3 for binding to the Reo3R. As indicated in Figure 12, $V_LC_9C_{16}$ peptide demonstrated higher affinity for the Reo3R than $V_LC_8C_{16}$ peptide or $V_LC_{10}C_{16}$ peptide. When compared with linear $V_L$ peptide (Figure 11), $V_LC_9C_{16}$ peptide demonstrated 40 fold higher affinity for the Reo3R, and similar affinity to dimeric $V_L$SH peptide.

This confirms that cyclic analogs of $V_L$ peptide demonstrate higher affinity of binding to the Reo3R than the linear peptide analogs. This strategy should be applicable to peptides derived from other antibody variable regions, and defines an overall strategy for determining the optimal conformation for binding of these peptides.

## 7. EXAMPLE: DESIGN OF IMMUNOGENIC HUMAN IMMUNODEFICIENCY VIRUS PEPTIDE

The putative CD4 binding domain of the human molecule (Lasky et al., 1987, Cell 50:975-985) was modelled after an immunoglobulin variable region. A topography of the purported gp120 binding site for CD4 was obtained based upon a comparative modelling approach utilizing sequence homology with the immunoglobulin superfamily (Kieber-Emmons et al., 1989, Biochem. Biophys. Acta 989:281-300). Molecular modeling of the 415-456 region of gp120 suggested that a disulfide bond can be formed by the cysteine residues at positions 418 and 445. The model indicates that the residues 421-438 define a central turn of the domain that is surface-exposed for possible interaction with CD4. Monoclonal antibodies that map to the 421-437 region have recently been shown to have anti-HIV neutralizing ability (Sun et al., 1989, J. Virol. <u>63:</u> 3579-3585). The 421-438 linear peptide termed B138 (Table I) was synthesized, purified, and assayed for its immunoreactivity with human sera and its ability to induce an anti-HIV response.

We have assessed the reactivity of linear B138 with sera (1:4 dilution) from healthy HIV-seronegative and seropositive study subjects. Low levels of reactivity to B138 were detected using sera from nineteen HIV-seropositive, asymptomatic individuals, with only 2 of 18 (11%) generating levels of anti-B138 antibodies that were significantly different (p<0.003) from HIV-seronegative controls. Thirty-three percent (7 of 21) of ARC patients and 10% (2 of 20) of AIDS patients had significant levels of antibodies that bound B138. Only one HIV-infected individual, an ARC patient, had levels of anti-B138 antibodies that were significantly different from HIV-seronegative individuals at 1:32 dilutions of serum (p<0. 003). These results demonstrate that HIV-1 does not stimulate the production of antibodies that bind B138 in most infected individuals. Furthermore, in the few individuals that had detectable levels of anti-B138 antibodies, these antibodies occurred at relatively low titers and did not correlate with neutralizing or protective activities.

The immunogenicity of B138 was determined by subcutaneous immunization of mice and rabbits. Mouse antisera to B138 was shown to preferentially bind to HIV$_{HTLVIIB}$-infected H9 cells as assessed by flow cytometry analysis. Rabbit anti-B138 sera could immunoprecipitate purified recombinant glycosylated gp120 as assessed by radiolabelled immunoprecipitations albeit weakly.

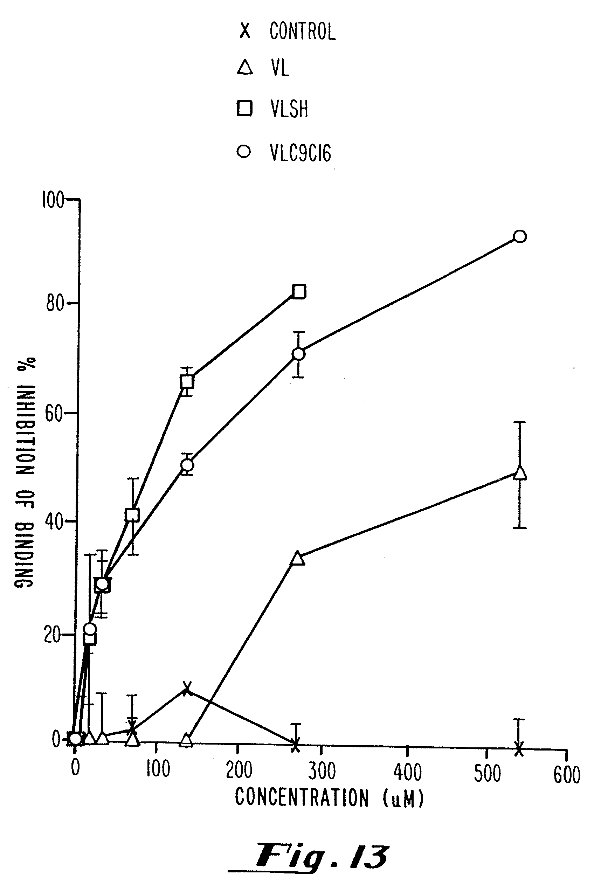

However, the immunogenicity of B138 increased dramatically when the B138 was made into the cyclic peptide, 1005-45 (Table II) by cysteine bond formation. Molecular dynamic calculations of the linear 421-438 segment and the cyclic 418-445 fragment suggested that the conformational populations available to the 421-438 region under the cyclic constraints were restricted and more similar to our predicted geometry of the gp120 putative CD4 binding epitope. Radiolabelled immunoprecipitations (RIPS) of rabbit anti-sera against the cyclic peptide showed significant specificity towards recombinant glycosylated gp120 (Figure 13). The reactivity is similar to that of the immunoreactivity of polyclonal human HIV positive sera reactive with gp120. Infectivity assays with rabbit anti-B138 and rabbit anti-1005/45 sera showed that both peptides could elicit neutralizing antibodies.

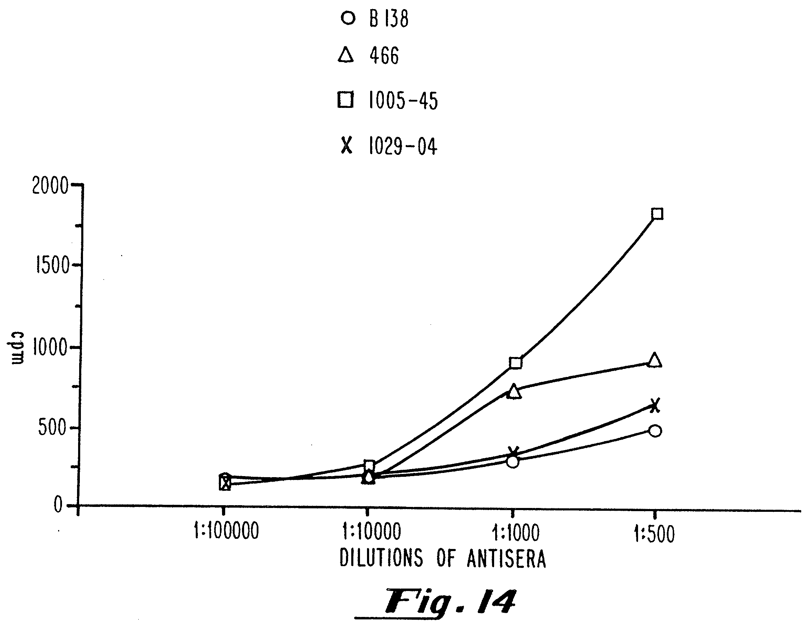

Figure 14 shows the results of experiments in which rabbits were immunized with various gp120-der peptides (see Table II) conjugated to Keyhole limpet hemocyanin (KLH) by glutaraldehyde fixation. Antisera was obtained and tested for binding to recombinant HIV-1 gp120 peptide-coated radioimmunoassay plates (using 1 µg/ml gp120 at 50 µl/well, in 0.1 M NaHCO$_3$ and incubating overnight at 4°C). The indicator antibody was $^{125}$I-labelled anti-rabbit immunoglobulin antibody. Figure 14 indicates that the highest levels of bound antibody were associated with antisera produced by animals immunized with 1005-45 cyclic peptide as compared to lower levels of bound antibody associated with antisera produced by animals immunized with linear B138. Higher bound levels of antibody could result from a generally greater immune response and/or the production of antibodies that have a higher affinity for gp120.

TABLE II

| Peptide Number | Sequence |
|---|---|
| 466 | FRPGGGDMRDNWSEL |
| 1005-45 | CRIKQFINMWQEVGKAMYAPPISGQIRC |
| B138 | KQFINMWQEVGKAMYAPP |

The present invention is not limited in scope by the embodiments disclosed in the examples which are intended as illustrations of a few aspects of the invention and any embodiments which are functionally equivalent are within the scope of this invention. Indeed, various modifications of the invention in addition to those shown and described herein will become apparent to those skilled in the art and are intended to fall within the scope of the appended claims.

**Claims**

**1.**   A method for producing a peptide which has biological activity comprising:

(i) identifying a region of a protein of interest which is homologous to a region of a member of the immunoglobulin superfamily of molecules;
(ii) synthesizing a peptide which comprises amino acid sequence found in the region of homology; and
(iii) modifying the synthetic peptide such that it assumes a three-dimensional conformation which is biologically active.

**2.**   The method according to claim 1 in which the member of the immunoglobulin superfamily is an immunoglobulin molecule.

3. The method according to claim 1 in which the member 3 of the immunoglobulin superfamily is a major histocompatibility complex molecule.

4. The method according to claim 1 in which the member of the immunoglobulin superfamily is-a T cell receptor molecule.

5. The method according to claim 1 in which the protein of interest is a viral protein.

6. The method according to claim 2 in which the protein of interest is a viral protein.

7. The method according to claim 3 in which the protein of interest is a viral protein.

8. The method according to claim 4 in which the protein of interest is a viral protein.

9. The method according to claim 5 in which the viral protein is a human immunodeficiency virus protein.

10. The method according to claim 6 in which the viral protein is a human immunodeficiency virus protein.

11. The method according to claim 7 in which the viral protein is a human immunodeficiency virus protein.

12. The method according to claim 8 in which the viral protein is a human immunodeficiency virus protein.

13. The method according to claim 9 in which the human immunodeficiency virus protein is gp120.

14. The method according to claim 10 in which the human immunodeficiency virus protein is gp120.

15. The method according to claim 5 in which the viral protein is a retrovirus protein.

16. The method according to claim 6 in which the viral protein is a retrovirus protein.

17. The method according to claim 7 in which the viral protein is a retrovirus protein.

18. The method according to claim 8 in which the viral protein is a retrovirus protein.

19. The method according to claim 1 in which the protein of interest is a cellular receptor for a virus.

20. The method according to claim 2 in which the protein of interest is a cellular receptor for a virus.

21. The method according to claim 3 in which the protein of interest is a cellular receptor for a virus.

22. The method according to claim 4 in which the protein of interest is a cellular receptor for a virus.

23. The method according to claim 19 in which the cellular receptor is CD4.

24. The method according to claim 20 in which the cellular receptor is CD4.

25. The method according to claim 21 in which the cellular receptor is CD4.

26. The method according to claim 22 in which the cellular receptor is CD4.

27. The protein prepared according to the method of claim 1.

28. The protein prepared according to the method of claim 2.

29. The protein prepared according to the method of claim 5.

30. The protein prepared according to the method of claim 9.

**31.** The protein prepared according to the method of claim 13.

**32.** The protein prepared according to the method of claim 15.

**33.** The protein prepared according to the method of claim 23.

**34.** The protein prepared according to the method of claim 24.

**35.** The protein prepared according to the method of claim 25.

**36.** The protein prepared according to the method of claim 26.

**37.** A method for designing a peptide having biological activity comprising

(i) identifying a portion of a protein of interest which functions as a complementarity determining region (CDR);

(ii) synthesizing a peptide which comprises a portion of the CDR identified in step (i); and

(iii) modifying the peptide such that it has biological activity.

**38.** The protein prepared according to the method of 37.

```
       383                                                          408
gp120  F Y C N S T Q L F N S T W F N S T W S T E G S N N T
Ig     Y Y C A R - N Y Y G S T W Y F D V W G A - G T T V T
       *     * *   * * * *       * * * *   - *     * * * *
       |_____|   |_____|   |_____|
          FR3H               CDR3H                      FR4H
```

```
       409                                                          441
gp120  E G S D T I T L P C R I K Q F I N M W Q E V G K A M Y A P P I S G
Ig         T I T - - C R A S Q S I N I W L A W Y Q Q K P E A P K L L
                        - - * * * * - - * * * - - *
           |_____|     |_____|   |_____|
              FR1L              CDR1L                    FR2L
```

```
       442                     455
gp120  Q I R C S N I T G L L L T
Ig         C S T D I N G Y F L F
               * *   *   * *   *
           |_____|
                  CDR3L
```

FIG. 1

# F I G. 2

EP 1 310 557 A1

```
L3T4   S Q L L A V T Q G K T L V L G K E G E S A E L P C E S S Q K
CD4    A L L P A A T Q G N K V V L G K K G D T V E L T C T A S Q K
IREI       D I Q M T Q S P S S L S A S V G D R V V T I T C Q A S Q D
IMPC       D I V M T Q S P S S L S V S A G E R R V V T M S C K S S Q S
IMCG         S A L T Q - P P S A S G S L G Q S V V T I S C T G T S S
IFBJ       E I V L T Q S P A I T A A S L G Q K V V T I T C S A S S S
IFB4       E S V L T Q - P P S A S G T P G Q R V V T I S C T G T S S
3FAB         S V L T Q - P P S V S G A P G Q R V V T I S C T G S S S

L3T4   - - - K I T V - - - F T W K F S D Q R K I L G Q H G K G V L
CD4    - - - K S I Q - - - F H W K N S N Q R I K I L G N Q G - S F L L
IREI   - - - I I K Y - - - L N W - - - Y Q Q T P - G K A P K - L L
IMCP   L L N S G N Q K N F L A W - - - Y Q Q K P - G Q P P K K - L L
IMCG   - - - D V G G Y N Y V S W - - - Y Q Q H A - G K A P K K - V I
IFBJ   - - - - - V S - - - L H W - - - Y Q Q K S - G T S P K K - P W
IFB4   N I G S I T - - - - V N W - - - Y Q Q L P - G M A P K K - L L
3FAB   N I G A G N H - - - V K W - - - Y Q Q L P - G T A P K K - L L

L3T4   I R G G S P S Q F D R F D S K K G A W E K G - S F P L I I
CD4    T K G P S K L N - D R A D S R R S L W D Q G G - N F P L I I
IREI   I Y E A S N L Q - A G V P S R F S G S G S G T D Y T F T I I
IMCP   I Y G A S T R E - S G V P D R F T G S G S G T D F T L T I I
IMCG   I Y E V N K R P - S G V P D R F S G S K S G N T A S L T I V
IFBJ   I Y E I S K L A - S G V P A R F S G S G S G T S Y S L T I I
IFB4   I Y R D A M R P - S G V P T R F S G S K S G T S A S L L A I I
3FAB   I F H N N A R F - S - V S - K - S G S - S - - - A T L A I I

L3T4   N K L K M E D S Q T Y I C
CD4    K N L K I E D S D T Y Y I C C
IREI   S S L Q P E D I A T Y Y I Y C
IMCP   S S V Q A E D L A V Y Y Y C C
IMCG   S G S Q A E D E A D Y Y Y C C
IFBJ   N T M E A E D A A E Y Y Y C C
IFB4   S G L E A E D E S D Y Y Y C C
3FAB   T G L Q A E D E A D Y Y Y C C
```

FIG.3

Fig. 4

_Fig. 5_

*Fig. 6*

*Fig. 7*

Fig. 8

*Fig. 9*

Fig. 10

Fig. II

Fig. 12

Fig. 13

*Fig. 14*

**European Patent Office**

**EUROPEAN SEARCH REPORT**

Application Number

EP 02 08 0338

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WILLIAMS W V ET AL: "SEQUENCES OF THE CELL-ATTACHMENT SITES OF REOVIRUS TYPE 3 AND ITS ANTI-IDIOTYPIC-ANTIRECEPTOR ANTIBODY MODELING OF THEIR THREE-DIMENSIONAL STRUCTURES" PROCEEDINGS OF THE NATIONAL ACADEMY OF SCIENCES OF THE UNITED STATES, vol. 85, no. 17, 1988, pages 6488-6492, XP002229610 1988 ISSN: 0027-8424 * the whole document * | 1,2,4-8, 19-22, 27-29, 37,38 | C12N15/12 C12N15/13 C07K16/00 C07K14/705 |
| X | WILLIAMS W V ET AL: "DEVELOPMENT OF BIOLOGICALLY ACTIVE PEPTIDES BASED ON ANTIBODY STRUCTURE" PROCEEDINGS OF THE NATIONAL ACADEMY OF SCIENCES OF USA, NATIONAL ACADEMY OF SCIENCE. WASHINGTON, US, vol. 86, no. 14, 1 July 1989 (1989-07-01), pages 5537-5541, XP000033900 ISSN: 0027-8424 * the whole document * | 1,2,4-8, 19-22, 27-29, 37,38 | |
| X | WO 89 10939 A (UNIVERSITY PATENTS, US) 16 November 1989 (1989-11-16) * the whole document * | 1,2,4-8, 19-22, 27-29, 37,38 | TECHNICAL FIELDS SEARCHED (Int.Cl.7) C12N C07K A61K |
| P,X | WO 91 04273 A (IDEC PHARMACEUTICALS CORP. , US) 4 April 1991 (1991-04-04) * page 9, line 6 - page 10, line 24; claims 1-25; example 3 * | 1-38 | |
| X | EP 0 328 403 A (UNITED BIOMEDICAL, US) 16 August 1989 (1989-08-16) * claims 1-4 * | 27-36 | |

-/--

The present search report has been drawn up for all claims

| Place of search | Date of completion of the search | Examiner |
|---|---|---|
| THE HAGUE | 3 February 2003 | Cupido, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

European Patent
Office

**Application Number**

EP 02 08 0338

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 89 03222 A (DANA FABER CANCER INSTITUTE) 20 April 1989 (1989-04-20)<br>* the whole document * | 27-36 | |

TECHNICAL FIELDS
SEARCHED      (Int.Cl.7)

The present search report has been drawn up for all claims

| Place of search | Date of completion of the search | Examiner |
|---|---|---|
| THE HAGUE | 3 February 2003 | Cupido, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 02 08 0338

03-02-2003

| Patent document cited in search report | | Publication date | Patent family member(s) | | Publication date |
|---|---|---|---|---|---|
| WO 8910939 | A | 16-11-1989 | AT | 140233 T | 15-07-1996 |
| | | | DE | 68926813 D1 | 14-08-1996 |
| | | | DE | 68926813 T2 | 17-07-1997 |
| | | | EP | 0370090 A1 | 30-05-1990 |
| | | | JP | 2504284 T | 06-12-1990 |
| | | | WO | 8910939 A1 | 16-11-1989 |
| | | | US | 6372884 B1 | 16-04-2002 |
| | | | US | 5637677 A | 10-06-1997 |
| WO 9104273 | A | 04-04-1991 | AU | 6523590 A | 18-04-1991 |
| | | | CA | 2066607 A1 | 23-03-1991 |
| | | | EP | 0493508 A1 | 08-07-1992 |
| | | | IE | 903433 A1 | 10-04-1991 |
| | | | WO | 9104273 A2 | 04-04-1991 |
| EP 0328403 | A | 16-08-1989 | AT | 154610 T | 15-07-1997 |
| | | | CA | 1341285 C | 14-08-2001 |
| | | | DE | 68928130 D1 | 24-07-1997 |
| | | | DE | 68928130 T2 | 20-11-1997 |
| | | | EP | 0328403 A2 | 16-08-1989 |
| | | | JP | 2022296 A | 25-01-1990 |
| | | | US | 5763160 A | 09-06-1998 |
| WO 8903222 | A | 20-04-1989 | US | 5109123 A | 28-04-1992 |
| | | | AU | 2614888 A | 02-05-1989 |
| | | | DK | 87490 A | 08-06-1990 |
| | | | EP | 0400010 A1 | 05-12-1990 |
| | | | ES | 2012129 A6 | 01-03-1990 |
| | | | GR | 1000850 B | 17-02-1993 |
| | | | JP | 3504234 T | 19-09-1991 |
| | | | NZ | 226475 A | 26-03-1991 |
| | | | WO | 8903222 A1 | 20-04-1989 |
| | | | ZA | 8807497 A | 26-07-1989 |

EPO FORM P0459